# EUROPEAN PATENT SPECIFICATION

(11) **EP 4 539 795 B1**
(45) Date of publication and mention of the grant of the patent: **18.02.2026**
(21) Application number: 22735383.6
(22) Date of filing: 17.06.2022
(51) Int. Cl.: A61F 13/15, A61F 13/551, B65D 65/46

(54) **A PACKAGE COMPRISING A BAG AND A STACK OF ABSORBENT ARTICLES**
VERPACKUNG MIT EINEM BEUTEL UND EINEM STAPEL SAUGFÄHIGER ARTIKEL
EMBALLAGE COMPRENANT UN SAC ET UNE PILE D'ARTICLES ABSORBANTS

(43) Date of publication of application: 23.04.2025
(73) Proprietor: Essity Hygiene and Health Aktiebolag, 405 03 Göteborg (SE)
(72) Inventor: THERSTOL, Hanna, 405 03 Göteborg (SE); DIVEKAR, Chetan, 405 03 Göteborg (SE)
(74) Representative: Nederlandsch Octrooibureau
(86) International application number: PCT/EP2022/066593
(87) International publication number: WO 2023/241811

(56) References cited:
- EP-A1- 3 685 812
- EP-A1- 3 865 421
- JP-A- 2017 159 559
- JP-A- 2021 035 754
- US-A1- 2022 031 531

## Description

### TECHNICAL FIELD

The disclosure relates to a package comprising a bag and a stack of absorbent articles arranged in the bag, such as disposable personal hygiene absorbent articles in the form of incontinent shields, sanitary napkins, panty liners, diapers, or the like.

### BACKGROUND

Disposable personal hygiene absorbent articles, for example absorbent pads such as incontinent shields or sanitary napkins, are typically provided in multipacks comprising one or more stacks of pads arranged in a plastic bag. Although plastic materials are versatile and offer a range of advantages such as low weight, high flexibility and easy sealing, plastic packaging materials may also be associated with disadvantages such as low degradability and recyclability, production of toxic gases during combustion, littering, etc. EP3865421A1 discloses a package comprising a bag and a stack of absorbent articles having a substantially rectangular shape, wherein the stack of absorbent articles is arranged in the bag. The bag is made of a paper material having a basis weight between 60 and 100 g/m2.

Consequently, there is currently a strive to replace plastic packaging by more sustainable and/or renewable alternatives, such as paper materials.

### SUMMARY

An object of the disclosure is to provide an, in at least some aspect, improved package comprising a bag and one or more stacks of absorbent articles. The object is achieved by a package according to claim 1.

Hence, a package comprising a bag and at least one stack of absorbent articles arranged in the bag is provided, wherein the bag is made of a paper material. The absorbent article may typically be a disposable personal hygiene absorbent article such as an incontinent shield, a sanitary napkin, a panty liner, a diaper, or the like. Preferably, the absorbent article may be an absorbent pad such as an incontinent shield, a sanitary napkin, or a panty liner. The at least one stack of absorbent articles may be at least one non-compressed stack of absorbent articles. Alternatively, the at least one stack of absorbent articles may be a slightly compressed stack of absorbent articles. A stack of absorbent articles comprises at least two absorbent articles. The stack may, purely by way of example, comprise 8 or 10 absorbent articles. However, the stack is not limited to 8 or 10 articles. Each stack may hence comprise more than 10 or less than 8 articles.

The bag has a sealed bottom, a sealed top, and a peripheral wall extending between the bottom and the top. The peripheral wall comprises a front wall, an opposite rear wall, and first and second side walls connecting the front and rear walls. The sealed top has been formed by adhering inner surface portions of the peripheral wall to one another.

The bag comprises an adhesion layer provided on the inner surface portions of the peripheral wall, said adhesion layer being used for adhering said inner surface portions to one another, wherein the adhesion layer has a basis weight within a range of 28-45 g/m². With a basis weight of at least 28 g/m², a sufficient amount of adhesive for ensuring a proper seal is ensured. Furthermore, by providing no more than 45 g/m², it can be ensured that the amount of adhesive is not excessive for the purpose of forming a firm bond.

The adhesion layer may preferably be provided only locally on the top portions to be adhered to one another, or alternatively over a larger portion of the inner surface or over substantially the entire inner surface of the bag. The adhesion layer may be applied as a continuous or discontinuous layer. When applied as a discontinuous layer, corner areas of the bag may be provided free from adhesive, which prevents the corners from being unintentionally adhered to one another during manufacture of the bag, i.e., prior to filling it with absorbent pads. The adhesion layer may in some embodiments extend over a closed peripheral inner portion of the inner surface of the bag. This ensures a good sealing of the bag, without any non-sealed portion(s) at the top of the bag.

Optionally, the adhesion layer has a basis weight of 30-40 g/m², preferably 34-38 g/m².

Optionally, a ratio between a basis weight of the paper material and the basis weight of the adhesion layer is within a range of 1.6-2.5, preferably 1.8-2.2.

Optionally, the adhesion layer is a hot-melt adhesion layer, i.e., a layer of a solid adhesive that has been applied in a molten state and hardened. During sealing, the hot-melt adhesive is melted again upon heating, as a result of which a sealing is formed upon cooling. Such a hot-melt adhesion layer may be used for almost instantaneous bonding. The hot-melt adhesion layer is a thermoplastic adhesive which is not water-soluble.

However, the adhesive of the adhesion layer is not limited to a hot melt adhesive, it may be any kind of adhesive suitable for forming a sealing in the present application. Other types of adhesives include adhesives which can dissolve in water. Such adhesives include starch based adhesives, polyvinyl alcohol based adhesives, and polyethylene oxide based adhesives. If the adhesive is not water-soluble, then water-dispersible adhesives may similarly be utilized. Suitable examples of water-dispersible adhesives include thermoplastic elastomer based adhesives and polyvinyl acetate based adhesives. Any suitable pressure sensitive adhesives may also be used.

The adhesive may be a bio-based adhesive.

Optionally, the paper material has a basis weight between 50 and 90 g/m² as measured according to the ISO 536 standard. A paper material having a basis weight within this range is sufficiently thick and strong to provide accurate protection for the absorbent articles within the bag against environmental pollution and can also withstand stresses arising during shaping of the bag and loading of absorbent articles into the bag. The paper material may be a single layer (one ply) or several layers.

By a paper material is herein to be understood a material produced by pressing together moist fibres, dewatering, and drying the fibres into preferably flexible sheets. The paper material may be an unbleached paper material or a bleached material. The fibres may be pulp fibres from chemical pulp, e.g. kraft, sulphate or sulphite, mechanical pulp, thermo-mechanical pulp, chemo-mechanical pulp and/or chemo-thermo-mechanical pulp, abbreviated as CTMP. Pulps derived from both deciduous (hardwood) and coniferous (softwood) may be used. The fibres may hence be natural fibres from wood, for example, cellulose-based fibres, bamboo based fibres, and the like. Natural fibres can also be fibres which are not wood, such as cotton, abaca, kenaf, sabai grass, flax, esparto grass, straw, jute hemp, bagasse, milkweed floss fibres, and pineapple leaf fibres. The paper material can be made of one kind of fibres or a mix of different fibres. The paper material may also be treated in a way to stand moisture better, for example with a plastic layer on the inside or the outside. The paper may be a smooth paper which has been calendared. The paper may be a paper with low porosity. The paper material may include recycled or virgin fibres or a combination thereof.

Optionally, the sealed top is formed by adhering the inner surface top portions of the peripheral wall to one another, such that:
- a first top portion of the front wall is adhered to an adjacent first top portion of the second side wall to form a first adhered portion having a first length,
- a second top portion of the front wall is adhered to an adjacent first top portion of the first side wall to form a second adhered portion having a second length,
- a first top portion of the rear wall is adhered to an adjacent second top portion of the second side wall to form a third adhered portion having a third length,
- a second top portion of the rear wall is adhered to an adjacent second top portion of the first side wall to form a fourth adhered portion having a fourth length, and
- a third top portion of the front wall is adhered to a third top portion of the rear wall to form a fifth adhered portion having a fifth length.

Optionally, the fifth length is smaller than each one of the first, second, third, and fourth lengths.

The fifth adhered portion herein connects the first and third adhered portions, provided closer to the second side wall, to the second and fourth adhered portions, provided closer to the first side wall. The first and third adhered portions hence form a first pair of wings, or fins, and the second and fourth adhered portions forms a second pair of wings, or fins. By making the fifth length smaller than the first, second, third, and fourth lengths, a bag is achieved which may easily be opened by manually pulling on opposite adhered portions, such as on the first and fourth adhered portions or on the second and third adhered portions. With the relatively small length of the fifth adhered portion, the pulling force may usually be sufficient to pull the joined portions of the front and rear walls apart so as to open the bag.

Preferably, none of the adhered portions is adhered to another one of the adhered portions. Further preferably, none of the adhered portions is adhered to an outer surface portion of the peripheral wall. In this way, no glue or adhesive must be provided on any outer surface portion of the peripheral wall for the purpose of adhering the wings within the first or second pair of wings to one another, or for adhering any one of the wings to the peripheral wall. By not being adhered to any outer surface portion of the peripheral wall, the adhered portions form suitable gripping members and opening of the bag as described above is facilitated.

Optionally, the fifth length is less than 75% of a shortest one of the first, second, third, and fourth lengths, preferably less than 65%. The fifth length may be less than 75%, or less than 65%, of each one of the first, second, third, and fourth lengths.

Optionally, the fifth length is within a range of 1-30 mm. Depending on the dimensions of the bag, this fifth length may in some embodiments be 1-8 mm, such as within a range of 2-6 mm, or 3-4 mm. In some embodiments, the fifth length may be within a range of 10-30 mm, such as 10-20 mm.

Optionally, the first, second, third, and fourth lengths are equal. Hence, the top is mirror symmetric with respect to at least two symmetry planes. "Equal" is herein to be understood as equal within manufacturing tolerances.

Optionally, a total length of the top, i.e., a sum of the first, second and fifth lengths, is equal to a total length of the bottom as measured in the same direction. "Equal" is herein to be understood as equal within manufacturing tolerances.

The package may have the basic shape of a cuboid. The bottom may be rectangular and have a width of 70-100 mm as measured between the front and rear walls, and a length of 80-180 mm, preferably 80-110 mm, as measured between the first and second side walls, wherein the length is preferably larger than the width. The package may have a filling height of 150-170 mm as measured from the bottom to the top, excluding the sealed top portion of the bag, for a bag with two rows of stacked absorbent articles on top of each other. However, the package may have a filling height of 70-100 mm as measured from the bottom to the top, excluding the sealed top portion of the bag, for a bag with one stack of absorbent articles. The bag may preferably be dimensioned so that the at least one stack of absorbent articles fits snuggly within the bag.

Optionally, a stacking direction of each one of the at least one stack of absorbent articles is perpendicular to the first and second side walls of the bag. Hence, within each one of the at least one stack of absorbent articles, the absorbent articles are stacked with side surfaces thereof facing one another and being aligned with the first and second side walls of the bag. Alternatively, the stacking direction of each one of the at least one stack of absorbent articles may be perpendicular to the front and rear walls of the bag.

Optionally, the peripheral wall of the bag comprises an overlap seam extending from the bottom of the bag to the top of the bag, wherein the overlap seam is formed at the front wall or at the rear wall. The other three walls are free from seams. The overlap seam is preferably formed at the rear wall, close to one of the side walls. Hence, the bag may be formed from a single sheet of paper material.

Optionally, the bag comprises at least one weakening line for opening the bag. Such a weakening line facilitates opening of the bag.

Optionally, the at least one weakening line comprises a perforation line or a score line. Other frangible elements that permit a user to easily tear the package in specific locations so to gain access to the interior of the package where the personal hygiene absorbent articles are located can also be used instead of a weakening line. In specific embodiments, the frangible element is an open-ended arcuate line or a closed geometric shape, e.g., an oval or circle having a perforated perimeter. Such a line or shape may be located entirely within a single one of the walls or extend between at least two adjacent ones of the walls.

Optionally, the at least one weakening line is formed in or adjacent to at least one of said adhered portions, such as just below at least one of said adhered portions, as seen when the package is in an upright position with the bottom resting on a support surface.

Optionally, the bag comprises at least one notch for tearing open the bag. The notch may be a V-shaped or U-shaped cut-out, allowing a user to grab portions of the bag located on opposite sides of the notch to tear open the bag.

Optionally, the at least one notch is formed in at least one of the adhered portions or adjacent to at least one of the adhered portions, as seen when the package is in an upright position with the bottom resting on a support surface. Preferably, the notch may be located at a top of one of the first, second, third, and fourth adhered portions.

The personal hygiene absorbent articles may be used for the absorption of bodily exudates, such as blood, urine, sweat and faeces. These personal hygiene absorbent articles may be packaged individually in wrappers, so that the article remains clean and intact prior to use. However, it is also possible to have two or three or more personal hygiene absorbent articles in a wrapper, which maybe suitable to bring along and use during the day. The wrapper may be a wrap where the article is folded together with wrapper material before the side edges, i.e. the edge areas, are sealed or a kind of pouch where the folded article is inserted into the pouch before the pouch is being sealed. Individual packages facilitate hygienic carrying of single articles for future use, e.g. in a handbag. Further, the packaging units are often used both as a means for packaging an unused article and for disposal of the used article.

Optionally, each one of the at least one stack of absorbent articles comprises a plurality of folded and individually wrapped absorbent articles. Alternatively, each one of the at least one stack of absorbent articles may comprise a plurality of folded absorbent articles without individual wraps. In both cases, the absorbent articles may be folded once or twice such that each folded absorbent article is folded into two or three folding layers. The fold or folds may preferably be arranged in parallel with the peripheral wall of the bag, i.e., perpendicularly to the bottom of the bag. Alternatively, the fold or folds may be arranged in parallel with the bottom of the bag.

The folded absorbent articles may have a width of 110-130 mm and a length of 70-95 mm.

Optionally, a wrapper of each individually wrapped absorbent article comprises a sealed wrapper portion facing upward within the bag. Hence, a user may easily remove a wrapped absorbent article from the top of the bag by grabbing the sealed wrapper portion. The sealed wrapper portion of each individually wrapped absorbent article may be aligned with the first and second side walls of the bag.

Optionally, the sealed bottom of the bag is formed by folding and adhering a portion of the paper material to form a flat and rectangular bottom. The sealed bottom may advantageously be formed using overlap seams, providing a flat bottom. The bottom may preferably be a block bottom.

Optionally, the bag comprises an air displacement hole at or close to the bottom of the bag for allowing air to escape from the bag. The air displacement hole is particularly useful for allowing air to escape during filling of the bag, i.e., when loading the at least one stack of absorbent articles into the bag. The air displacement hole should preferably be located close to one of the side walls.

Optionally, the at least one stack of absorbent articles comprises two stacks of absorbent articles, preferably two parallel stacks of absorbent articles having a stacking direction perpendicular to the first and second side walls of the bag. The two stacks of absorbent articles may be a bottom stack located in the bottom of the bag and a top stack located in the top of the bag. In some embodiments, the at least one stack of absorbent articles may comprise more than two stacks of absorbent articles, such as four stacks of absorbent articles. For example, two bottom stacks and two top stacks may be provided, wherein a first bottom stack and a first top stack are located closer to the first side wall, and wherein a second bottom stack and a second top stack are located closer to the second side wall.

Optionally, the bag is opaque and the absorbent articles, such as a wrapper of individually wrapped absorbent articles, cannot be seen through the bag. Information printed on the package can thereby be made clearly visible to a user.

Further advantages and advantageous features of the invention are disclosed in the following description and in the dependent claims.

### BRIEF DESCRIPTION OF THE DRAWINGS

- Fig. 1: is a perspective view of a package according to an embodiment,
- Fig. 2: is a perspective view of a package according to another embodiment,
- Fig. 3: is a top view of the package in fig. 1,
- Fig. 4: is a top view of the package in fig. 1 in an open state,
- Fig. 5: is another perspective view of the package in fig. 1,
- Fig. 6: is a side view of the package in fig. 1,
- Fig. 7: schematically illustrates a folded and wrapped absorbent pad,
- Fig. 8: illustrates the absorbent pad from fig. 7,
- Fig. 9: illustrates a paper sheet used to form a package according to an embodiment of the disclosure, and
- Fig. 10: illustrates a paper sheet used to form a package according to another embodiment of the disclosure.

### DETAILED DESCRIPTION OF PREFERRED EMBODIMENTS

Various aspects of the disclosure will hereinafter be described in conjunction with the appended drawings to illustrate.

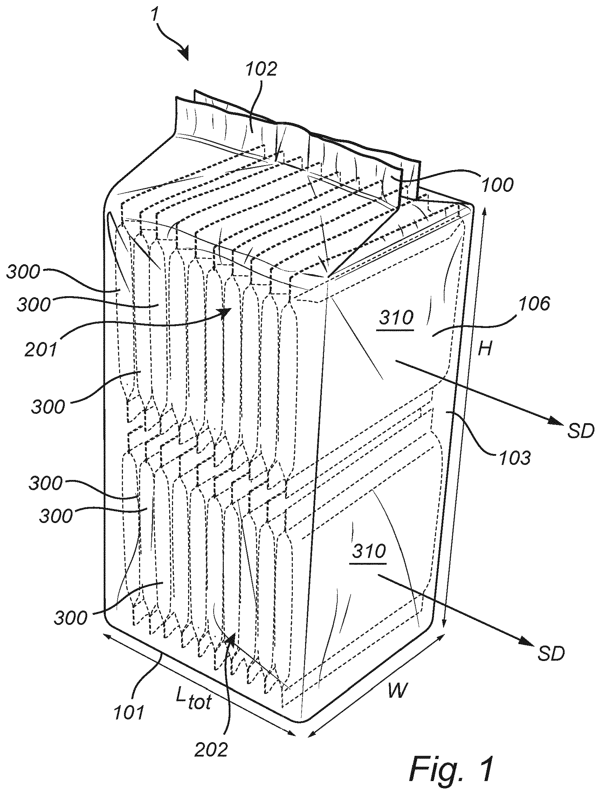

A package 1 according to embodiments of the disclosure is schematically illustrated in Figs. 1-6. In the illustrated embodiments, the package 1 comprises a bag 100 and two stacks 201, 202 of absorbent articles 300 in the form of absorbent pads 300 arranged in the bag 100. One stack 201 is arranged on top of the other stack 202. The bag 100 is made of a paper material and has a sealed bottom 101, a sealed top 102, and a peripheral wall 103 extending between the bottom 101 and the top 102. The peripheral wall 103 comprises a front wall 104, an opposite rear wall 105, and first and second side walls 106, 107 connecting the front wall 104 and the rear wall 105.

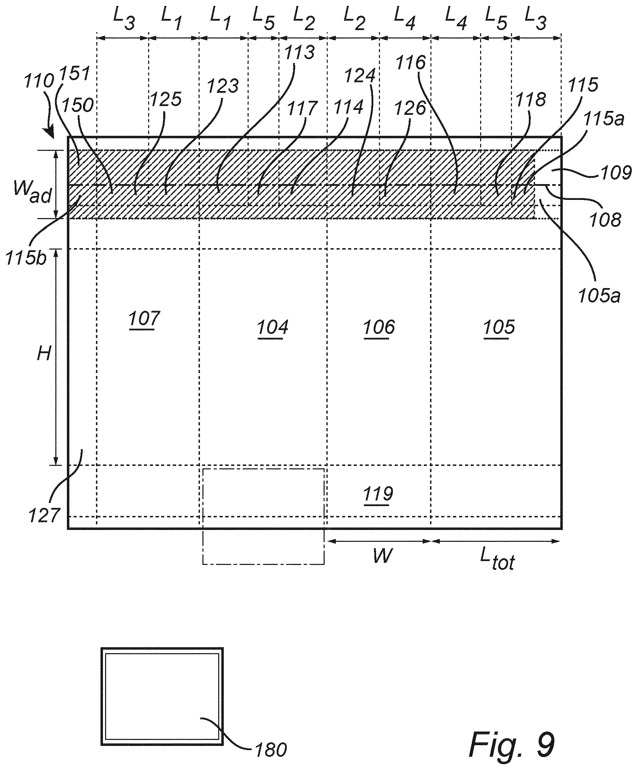

The bag 100 is formed from a single sheet 110 of rectangular paper as illustrated in Fig. 9, which is folded, glued, and cut to form the bag 100. Once the bottom 101 and the peripheral side wall 103 of the bag 100 have been formed and sealed, and the bag 100 has been filled with the absorbent pads 303, the bag 100 is folded and sealed at the top 102 and a superfluous top portion 109 above a cutting line 108 as illustrated in Fig. 9 is cut away.

The sealed top 102 of the bag 100 has been formed by adhering inner surface top portions of the peripheral wall 103, i.e., of the paper sheet 110, to one another so as to form wings, or fins. The inner surface top portions are illustrated in Fig. 9 and comprise: a first top portion 113 of the front wall 104 and an adjacent first top portion 123 of the second side wall 107; a second top portion 114 of the front wall 104 and an adjacent first top portion 124 of the first side wall 106; a first top portion 115 of the rear wall 105 and an adjacent second top portion 125 of the second side wall 107; a second top portion 116 of the rear wall and an adjacent second top portion 126 of the first side wall 106; a third top portion 117 of the front wall 104, located between the first and second top portions 113, 114 of the front wall 104; and a third top portion 118 of the rear wall 105, located between the first and second top portions 115, 116 of the rear wall 105.

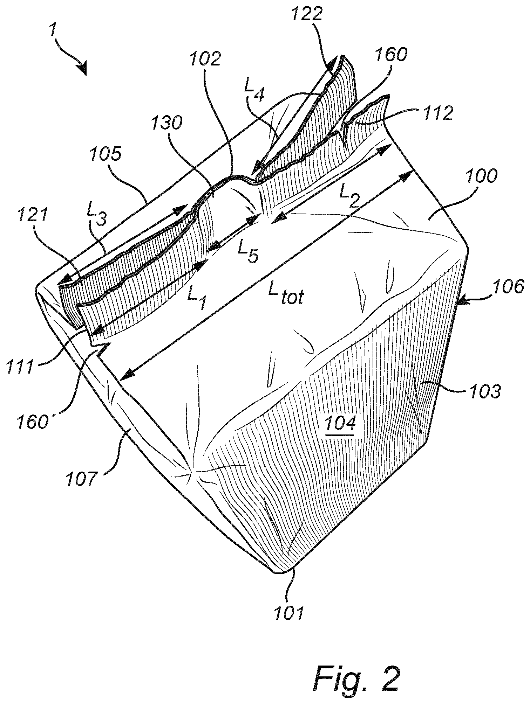

The first top portion 113 of the front wall 104 is adhered to the adjacent first top portion 123 of the second side wall 107 to form a first adhered portion 111 having a first length L₁ as shown in Fig. 2 and Fig. 9. The second top portion 114 of the front wall 104 is adhered to the adjacent first top portion 124 of the first side wall 106 to form a second adhered portion 112 having a second length L₂. The first top portion 115 of the rear wall 105 is adhered to the adjacent second top portion 125 of the second side wall 107 to form a third adhered portion 121 having a third length L₃. The second top portion 116 of the rear wall 105 is adhered to the adjacent second top portion 126 of the first side wall 106 to form a fourth adhered portion 122 having a fourth length L₄. The third top portion 117 of the front wall 104 is adhered to the third top portion 118 of the rear wall 105 to form a fifth adhered portion 130 having a fifth length L₅. The fifth length L₅ is smaller than each one of the first length L₁, the second length L₂, the third length L₃, and the fourth length L₄. The lengths L₁, L₂, L₃, L₄ and L₅ are all measured in a direction parallel with the front wall 104, with the adhered portions 111, 112, 121, 122, 130 stretched out to be as long as possible.

In the illustrated embodiments, the first length L₁, the second length L₂, the third length L₃, and the fourth length L₄ are all equal, within manufacturing tolerances. The fifth length L₅ is herein less than less than 75% of each one of the first length L₁, the second length L₂, the third length L₃, and the fourth length L₄, preferably less than 65%, or less than 60%. The first length L₁, the second length L₂, the third length L₃, and the fourth length L₄ may be between 30-50 mm, preferably 35-45 mm. The fifth length L₅ may be 1-30 mm, more preferably 3-25 mm. A total length Lₜₒₜ of the front wall 104 and/or back wall 105 of the bag 100, as measured between the side walls 106, 107 at a bottom of the package 1, is essentially equal to the sum of the first length L₁, the second length L₂, and the fifth length L₅ and/or the sum of the third length L₃, the fourth length L₄ and the fifth length L₅.

In the illustrated embodiment, the first length L₁ is 39 mm and the fifth length L₅ is 22 mm, i.e., 56% of the first length L₁. In the illustrated embodiment the bag 100 has a total length Lₜₒₜ of 100 mm as measured between the side walls 106, 107, and a total width W as measured between the front and rear walls 104, 105 of 78 mm. A filling height H of the bag 100 is in the illustrated embodiment 163 mm.

In another embodiment, the first length L₁ is 45 mm and the fifth length L₅ is 10 mm, i.e., 22% of first length L₁. The bag 100 has a total length Lₜₒₜ of 100 mm as measured between the side walls 106, 107, and a total width W as measured between the front and rear walls 104, 105 of 88 mm. A filling height H of the bag 100 is in the illustrated embodiment 165 mm.

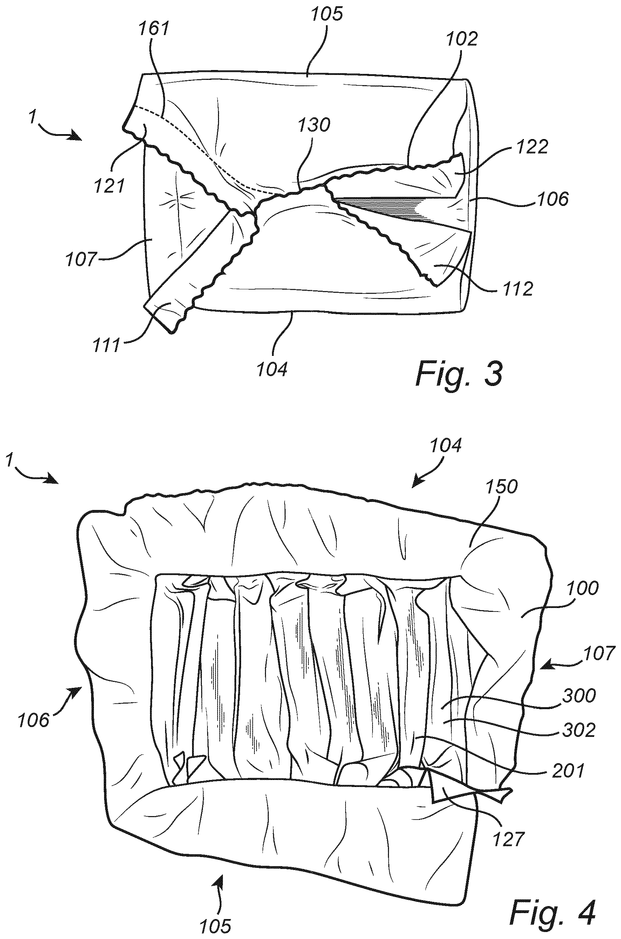

As best seen in Fig. 3, the fifth adhered portion 130 connects the first and third adhered portions 111, 121, provided closer to the second side wall 107, to the second and fourth adhered portions 112, 122, provided closer to the first side wall 106. The first and third adhered portions 111, 121 hence form a first pair of wings and the second and fourth adhered portions 112, 122 forms a second pair of wings. None of the adhered portions 111, 112, 121, 122, 130 is adhered to another one of the adhered portions 111, 112, 121, 122, 130 by e.g. an overlap seam or a butt seam. Furthermore, none of the adhered portions 111, 112, 121, 122, 130 is adhered to an outer surface portion of the peripheral wall 103. Since they are not adhered to any outer surface portion of the peripheral wall, the first, second, third and fourth adhered portions 111, 112, 121, 122, form suitable gripping members for opening of the bag.

The bag 100 comprises an adhesion layer 150 provided on the inner surface portions 113, 114, 115, 116, 117, 118, 123, 124, 125, 126 of the peripheral wall 103, as shown in Fig. 9, wherein the adhesion layer 150 is shown as a shaded area. The adhesion layer 150 is used for adhering the inner surface portions to one another to form the butt seams as described above. The adhesion layer has a basis weight of 28-45 g/m², preferably 30-40 g/m², or more preferably 34-38 g/m² and be a hot-melt adhesion layer.

The basis weight of the adhesion layer may be determined by weighing a first sample of the sheet 110 on which the adhesion layer 150 is provided, and a second sample of the same size on which no adhesion layer is provided. Alternatively, the adhesion layer may be removed from the first sample, whereafter the first sample is weighed again. A total weight of the adhesion layer provided on the first sample is thereby obtained as the difference in weight between the samples with and without adhesion layer. The weight of the adhesion layer is divided by a surface area of the first sample to obtain the basis weight.

The adhesion layer 150 can, for example, comprise or consist of a hot melt adhesive. However, the adhesive is not limited to a hot melt adhesive, it may be any kind of adhesive suitable for the application. Other kind of adhesives can be adhesives which can dissolve in water. Such adhesives include starch based adhesives, polyvinyl alcohol based adhesives, and polyethylene oxide based adhesives. If the adhesive is not water-soluble, then water-dispersible adhesives may similarly be utilized. Suitable examples of water dispersible adhesives include thermoplastic elastomer based adhesives and polyvinyl acetate based adhesives. Any suitable pressure sensitive adhesives may also be used.

The adhesion layer 150 is herein partially provided on the superfluous top portion 109 which is cut away after sealing. The adhesion layer 150 may prior to adhesion, folding and cutting have a width W_{ad} of 30-60 mm as measured in a height direction of the bag 100.

The sealing at the top 102 of the bag 100 can be accomplished by pressing two seal bars against each other, with the folded top portions 113, 114, 115, 116, 117, 118, 123, 124, 125, 126 of the bag 100 comprising the adhesion layer 150 inserted between the seal bars. Thereby, the hot melt adhesive applied to the inside of the bag 100 melts and the folded top portions 113, 114, 115, 116, 117, 118, 123, 124, 125, 126 attach to each other. The sealing width accomplished by the sealing bars may be 3-10 mm measured in a height direction of the bag 100, more preferably 3-6 mm. After the bag 100 has been cut, the sealing width may be smaller or the same, depending on if the cutting line 108 along which the cut is made is above the seal or not.

The seal of the packages may have a tensile strength of at least 1 N per 25.4 mm. For example, each of the plurality of seals of the packages of the present disclosure may have a tensile strength of between 1 N per 25.4 mm to about 22 N per 25.4mm, more preferably from about 3 N per 25.4 mm to about 17 N per 25.4 mm. The tensile strengths of the seals mentioned herein can be determined by the tensile test method described in ASTM F88-21 as modified herein.

As used herein, a paper material is to be understood as a material produced by pressing together moist fibres, dewatering, and drying the fibres into preferably flexible sheets. The paper material used herein may have a basis weight between 50 and 90 g/m² as measured according to the ISO 536 standard. A ratio between the basis weight of the paper material used for the bag 100 and the basis weight of the adhesion layer 150 may be within a range of 1.6-2.5, preferably 1.8-2.2. For example, the paper material may have a basis weight of 70 g/m² and the adhesion layer 150, such as the hot-melt adhesion layer, may have a basis weight of approximately 36 g/m².

The paper material may have any one or more of following properties:
- a tensile strength measured according to the ISO 1924-3 standard in a machine direction between 4 and 10 kN/m, preferably between 6 and 8 kN/m, and in a cross direction between 2 and 6 kN/m, preferably between 3 and 5 kN/m;
- a tear index measured according to the ISO 1974 standard in a machine direction between 8 and 14 mNm²/g, and in a cross direction between 8 and 15 mNm²/g;
- a tear strength measured according to the ISO 1974 standard in a machine direction between 400 and 1300 mN, and in a cross direction between 450 and 1400 mN,
- a stretch measured according to the ISO 1924-3 standard in a machine direction between 1 and 6 %, and in a cross direction between 5 and 12 %.

As illustrated in Fig. 2, the bag 100 may in certain embodiments comprise a first notch 160 for tearing open the bag 100. The first notch 160 is herein formed at a top edge of the second adhered portion 112, although it may alternatively be provided in any one of the first, third and fourth adhered portions 111, 121, 122. As further illustrated, the bag 100 may alternatively or additionally comprise a second notch 160' for tearing open the bag 100, the second notch 160' being formed just below one of the adhered portions, herein below the first adhered portion 111. In alternative embodiments, the notches 160, 160' may be replaced or complemented by a weakening line 161 in the form of a perforation line or a score line such as illustrated in Fig. 3. The weakening line may e.g. be located below one or more of the adhered portions 111, 112, 121, 122, such as below the third adhered portion 121 as illustrated in Fig. 3. In yet other embodiments, no notch or weakening line is provided. In those cases, the bag 100 may be opened by, e.g. manually pulling on opposite adhered portions. The package 1 in an open state is illustrated in Fig. 4.

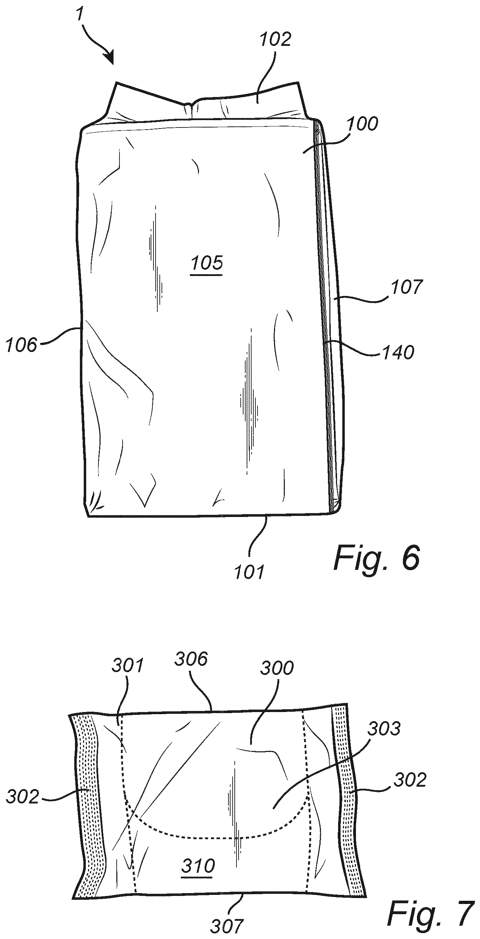

The rectangular sheet 110 is in Fig. 9 illustrated with dashed folding lines along which the sheet 109 is folded to form the bag 100. To form the peripheral side wall 103, an edge portion 105a of the rear wall 105 is overlapped with a rectangular seam portion 127 located next to the second side wall 107 on the rectangular sheet 110. After folding the sheet 110, the edge portion 105a of the rear wall 105 overlaps the seam portion 127 and an overlap seam 140 is formed. Hence, the seam portion 127 forms part of the rear wall 105 after folding and sealing. The overlap seam 140 extends from the bottom 101 of the bag 100 to the top 102 of the bag 100 as illustrated in Fig. 6. An adhesive may be used to form the overlap seam 140. The adhesive may be of the same type as used for the adhesion layer 150, or of a different type. The adhesive may, e.g., be a hot-melt adhesive, i.e., a thermoplastic adhesive which is not water-soluble. However, the adhesive is not limited to a hot melt adhesive, it may be any kind of adhesive suitable for the application. Other kind of suitable adhesives can be adhesives which can dissolve in water. Such adhesives include starch based adhesives, polyvinyl alcohol based adhesives, and polyethylene oxide based adhesives. If the adhesive is not water-soluble, then water-dispersible adhesives may similarly be utilized. Suitable examples of water dispersible adhesives include thermoplastic elastomer based adhesives and polyvinyl acetate based adhesives. Any suitable pressure sensitive adhesives may also be used.

The seam portion 127 illustrated in Fig. 9 comprises an end portion 151 of the adhesion layer 150, provided on an inner surface portion 115b which forms part of the first top portion 115 of the rear wall 105 after the overlap seam 140 has been formed. The inner surface portion 115b together with an adjacent inner surface portion 115a hence forms the first top portion 115 of the rear wall 105. The seam portion 127 is located inside of the edge portion 105a of the rear wall 105 after the overlap seam 140 has been formed, i.e., on the inside of the package 1. The edge portion 105a of the rear wall 105 is formed without adhesion layer 150. The edge portion 105a of the rear wall may herein have a width of about 25 mm.

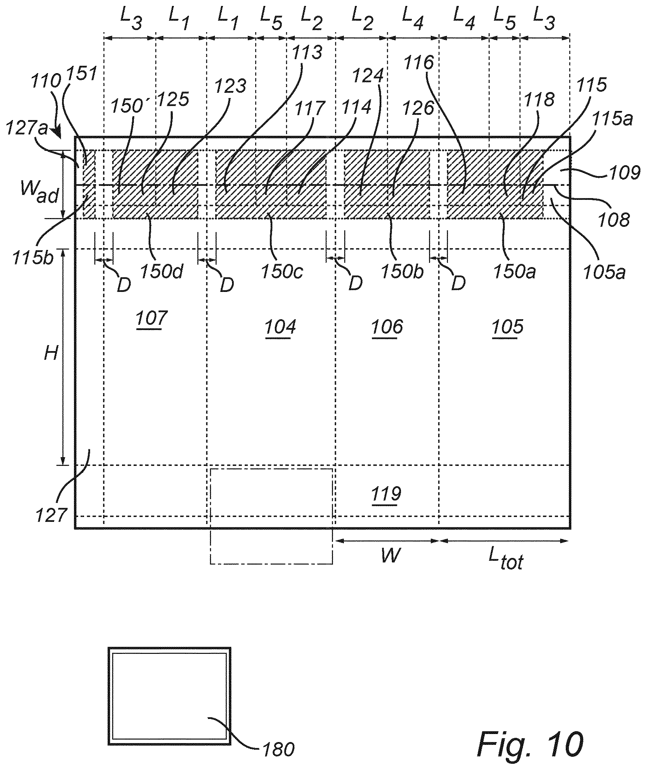

The adhesion layer 150 shown in Fig. 9 is a continuous adhesion layer. However, an alternative is shown in Fig. 10, wherein the adhesion layer 150' is a discontinuous adhesion layer comprising a first layer portion 150a provided on the rear wall 105, a second layer portion 150b provided on the first side wall 106, a third layer portion 150c provided on the front wall 104, a fourth layer portion 150d provided on the second side wall 107, and an end layer portion 151 provided on the seam portion 127. Between each two adjacent layer portions 150a-d, 151, an area free of adhesive is provided. The dashed folding lines are located in the areas free of adhesive. A distance D between each two adjacent layer portions 150a-d, 151 may be in a range of 5-15 mm, preferably 8.5-11.5 mm, such as about 10 mm. This prevents the corners from becoming adhered during manufacturing of the bag 100, prior to filling it with the absorbent pads 300. In the embodiment illustrated in Fig. 10, the edge portion 105a of the rear wall 105 is also free of adhesive, as well as an edge portion 127a of the seam portion 127. The adhesive free edge portion 127a of the seam portion 127 may herein have a width of about 5 mm.

The bag 100 may further comprise an air displacement hole (not shown) located at or close to the bottom 101 of the bag 100 for allowing air to escape from the bag 100, such as when loading the pads 300 into the bag 100 and for reducing tension that may arise as the bag 100 is subjected to pressure. The air displacement hole may be located close to an edge between any two adjacent walls. It may preferably be located at one of the side walls 106, 107, adjacent to the bottom 101. It may alternatively be formed as a non-sealed lower portion of the overlap seam 140.

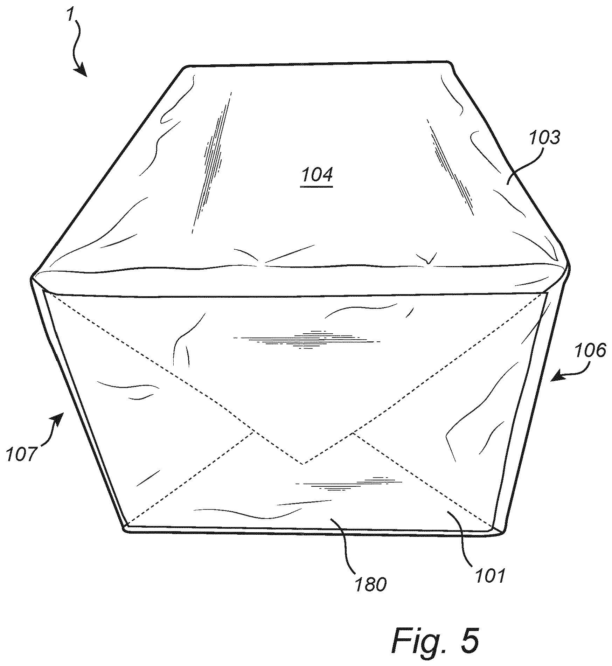

An example configuration of the bottom 101 is illustrated in Fig. 5. The sealed bottom 101 of the bag 100 is a block bottom, herein formed by folding a rectangular lower portion 119 of the sheet 110, and adhering the resulting folded portions to each other to form a flat and rectangular bottom 101. The sealed bottom 101 is formed using overlap seams to provide a flat bottom. A rectangular patch 180, forming a bottom label, is glued as a cover onto the bottom 101.

All external sides of the bag 100 may be covered with a layer of varnish, protecting any printed portions of the bag 100. Printed portions may be provided on the peripheral wall 103 as well as on the top 102 and/or on the bottom label.

Since the absorbent pads 300 are non-compressed or only slightly compressed, the package 1 has relatively sharp 90° edges. The front and rear walls 104, 105 as well as the first and second side walls 106, 107 are relatively flat. The length Lₜₒₜ of the package 1 as well as the width W are therefore close to constant along the height H of the package, as can be gleaned from Fig. 1. A maximum total length Lₜₒₜ of the package 1, as measured anywhere along the stacking height H, preferably differs by 10 mm or less, more preferably by 8 mm or less, from a minimum total length Lₜₒₜ as measured at the sealed bottom 101 or at the sealed top 102. In other words, the side walls 106, 107 do not bulge out significantly.

Two stacks 201, 202 of folded and individually wrapped absorbent pads 300, such as disposable personal hygiene absorbent pads in the form of incontinent shields, sanitary napkins, panty liners, or the like, are in the illustrated embodiments arranged in the bag 100.

Figs. 7-8 illustrate an absorbent pad 300 in the form of an incontinent shield according to an example embodiment. Such incontinent shields are, e.g., described in WO2017086849. The absorbent pad 300 may, purely by way of example, comprise a liquid pervious topsheet 309 bonded to an underlying acquisition or distribution layer (not shown), a liquid impermeable backsheet (not shown), and an absorbent core (not shown) sandwiched between the acquisition or distribution layer and the liquid impermeable backsheet. However, many different configurations are possible.

The illustrated absorbent pad 300 is a wing-less incontinent shield that is intended to be adhesively attached to an underwear of a user, for example by means of adhesive that is provided on an outer side of the backsheet for fastening the absorbent pad 300 to said underwear. However, the disclosure is equally applicable to wing-type absorbent pads, where laterally protruding wings are intended to be folded around the edge of the underwear of a user for fastening the absorbent pad 300 to said underwear.

The liquid permeable topsheet 309 can comprise a nonwoven material, e. g. spunbonded, meltblown, carded, hydroentangled, wetlaid, etc. which can be composed of natural fibres, such as woodpulp or cotton fibres, synthetic fibres, such as polyester, polyethylene, polypropylene, viscose, etc. or from a mixture thereof. The topsheet 309 material may further be composed of tow fibres, porous foams, apertured plastic films etc. The materials suited as topsheet materials should be soft and non-irritating to the skin and be readily penetrated by body fluid, e g urine or menstrual fluid, and display low rewetting properties. The topsheet 309 may comprise weldable fibres for enabling bonding to the underlying sheet by means of welding, such as ultrasonic welding. Alternatively, the topsheet 309 may be bonded to the underlying material with adhesive, or just placed on top of the underlying material.

The acquisition or distribution layer, if such a layer is used in absorbent pad 300, may comprise a material suitable for bonding to the topsheet 309. For example, if welding technology is used for bonding the topsheet 309 to the acquisition or distribution layer, the material of the acquisition or distribution layer should include at least partly weldable fibres. For example, the weldable fibres may polyethylene fibres coated with a layer of polypropylene. The acquisition or distribution layer may further include pure polyethylene fibres. In a specific example less than 30% of the fibres in the acquisition or distribution layer are polyethylene fibres and more than 70% of the fibres are welding fibres consisting of polyethylene fibres coated with polypropylene.

The absorbent core may comprise any conventional material suitable for absorbing discharged bodily wastes, such as cellulosic fluff pulp, tissue layers, highly absorbent polymers (superabsorbents), absorbent foam materials including hydrogel-foam material, absorbent nonwoven materials or the like. Similar to above, if welding technology is used for bonding the topsheet 309 directly to the absorbent core, the material of the absorbent core should include at least partly weldable fibres.

The liquid impermeable backsheet may comprise a thin plastic film, e. g. a polyethylene or polypropylene film, a nonwoven material coated with a liquid impervious material, a hydrophobic nonwoven material which resists liquid penetration or laminates of plastic films and nonwoven materials. The backsheet material may be breathable so as to allow vapour to escape from the absorbent core, while still preventing liquids from passing through the backsheet material.

In one embodiment, the absorbent pad 300 may be an absorbent pad sold commercially by Essity Hygiene and Health AB under the name TENA discreet Mini. The package 1 may then comprise two stacks 201, 202 with, purely by way of example, 8 or 10 absorbent pads 300 in each stack 201, 202.

In another embodiment, the absorbent pad 300 may be an absorbent pad sold commercially by Essity Hygiene and Health AB under the name TENA discreet Mini Plus.

The package 1 may then comprise two stacks 201, 202 with, purely by way of example, 8 or 10 absorbent pads 300 in each stack 201, 202.

Each absorbent pad 300 is in the illustrated embodiment provided within an individual wrapper 301, such as a thin sheet or similar, to which the absorbent pad 300 is releasably adhered before use. The sheet material for forming the wrapper 301 may be a single ply sheet of any suitable material known to the person skilled in the art, such as polyethylene or polypropylene film or nonwoven or paper. The wrapper 301 may also be a laminate comprising at least two distinct layers. Laminates suitable for packaging of hygiene articles are assumed to be known to the person skilled in the art.

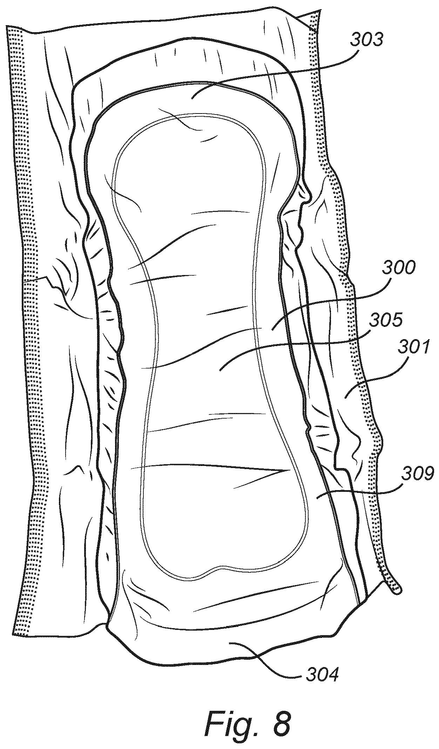

The absorbent pad 300 attached to the individual wrapper 301 is herein folded twice to form three folding layers, with a front portion 303 overlapping a rear portion 304, both the front and rear portions 303, 304 overlapping a centre portion 305. In the embodiment illustrated in Fig. 8, the pad 300 is folded together with the wrapper 301. Alternatively the pad may be pre-folded and inserted into the wrapper. Each individually wrapped absorbent pad 300 comprises two sealed wrapper portions 302 extending perpendicularly to opposite folded edges 306, 307 of the absorbent pad 300. One of the sealed wrapper portions 302 faces upward within the bag 100 as illustrated in Fig. 1 and Fig. 4. Hence, a user may easily remove a wrapped absorbent pad 300 from the opened top of the bag 100 by grabbing the sealed wrapper portion 302. However, the sealed wrapper portion 302 is not limited to be arranged facing upward with in the bag 100. It may alternatively face any one of the other surfaces of the bag 100.

The sealed wrap portions 302 may be sealed by an adhesive, for example a resealable adhesive or a non-resealable adhesive and/or sealed by means of ultrasonic welding or heat welding. By the term "resealable adhesive" is meant an adhesive that provides a non-permanent adhesive bond between two adherent surfaces, i.e. a bond that may be broken by applying a pulling force to the adherent, and recreated by applying a pressing force to the adherent. The resealable adhesive may be a pressure-sensitive hotmelt adhesive.

A stacking direction SD of each stack 201, 202 within the bag 100 is perpendicular to the first and second side walls 106, 107 of the bag 100, see Fig. 1. Hence, within each stack 201, 202, the folded and individually wrapped absorbent pads 300 are stacked with side surfaces 310 thereof facing one another. The side surfaces 310 are aligned with the first and second side walls 106, 107 of the bag 100.

To manufacture a package 1 according to the disclosure, the at least one stack 201, 202 of absorbent articles 300 is arranged in a bag 100 as described above, wherein the bag has a closed bottom 101, an open top 102, and a peripheral wall 103 between the bottom 101 and the top 102. The inner surface top portions of the peripheral wall 103 provided with the adhesion layer 150 are thereafter adhered to one another as described above to obtain a closed top 102.

Moreover, many modifications may be made to adapt a particular situation or material to the teachings of the present disclosure without departing from the essential scope thereof. Therefore, it is intended that the present disclosure not be limited to the particular examples illustrated by the drawings and described in the, but that the scope of the present disclosure will include any embodiments falling within the foregoing description and the appended claims.

Reference signs mentioned in the claims should not be seen as limiting the extent of the matter protected by the claims, and their sole function is to make claims easier to understand.

## Claims

1. A package (1) comprising a bag (100) and at least one stack of absorbent articles (200) arranged in the bag (100), the bag (100) being made of a paper material,
wherein the bag (100) has a sealed bottom (101), a sealed top (102), and a peripheral wall (103) extending between the bottom (101) and the top (102), wherein the top (101) has been formed by adhering inner surface top portions (113, 114, 115, 116, 117, 118, 123, 124, 125, 126) of the peripheral wall (103) to one another,
wherein the bag (100) comprises an adhesion layer (150) provided on the inner surface portions (113, 114, 115, 116, 117, 118, 123, 124, 125, 126) of the peripheral wall (103), said adhesion layer (150) being used for adhering said inner surface portions (113, 114, 115, 116, 117, 118, 123, 124, 125, 126) to one another,
wherein the adhesion layer (150) has a basis weight within a range of 28-45 g/m².

2. The package according to claim 1, wherein the basis weight of the adhesion layer (150) is within a range of 30-40 g/m², preferably 34-38 g/m², and/or wherein a ratio between a basis weight of the paper material and the basis weight of the adhesion layer (15) is within a range of 1.6-2.5, preferably 1.8-2.2.

3. The package according to any one of the preceding claims, wherein the adhesion layer (150) is a hot-melt adhesion layer.

4. The package according to any one of the preceding claims, wherein the paper material has a basis weight between 50 and 90 g/m² as measured according to the ISO 536 standard.

5. The package (1) according to any one of the preceding claims, wherein the peripheral wall (103) comprises a front wall (104), an opposite rear wall (105), and first and second side walls (106, 107) connecting the front and rear walls (104, 105), wherein the sealed top (102) has been formed by adhering the inner surface top portions (113, 114, 115, 116, 117, 118, 123, 124, 125, 126) of the peripheral wall (103) to one another such that:
- a first top portion (113) of the front wall (104) is adhered to an adjacent first top portion (123) of the second side wall (107) to form a first adhered portion (111),
- a second top portion (114) of the front wall (104) is adhered to an adjacent first top portion (124) of the first side wall (106) to form a second adhered portion (112),
- a first top portion (115) of the rear wall (105) is adhered to an adjacent second top portion (125) of the second side wall (107) to form a third adhered portion (121),
- a second top portion (116) of the rear wall (105) is adhered to an adjacent second top portion (126) of the first side wall (106) to form a fourth adhered portion (122), and
- a third top portion (117) of the front wall (104) is adhered to a third top portion (118) of the rear wall (105) to form a fifth adhered portion (130).

6. The package (1) according to claim 5, wherein first adhered portion (111) has a first length (L₁), the second adhered portion (112) has a second length (L₂), the third adhered portion (121) has a third length (L₃), the fourth adhered portion (122) has a fourth length (L₄), and the fifth adhered portion has a fifth length (L₅), wherein the fifth length (L₅) is smaller than each one of the first (L₁), second (L₂), third (L₃), and fourth (L₄) lengths.

7. The package according to claim 6, wherein the fifth length (L₅) is less than 75% of a shortest one of the first (L₁), second (L₂), third (L₃), and fourth (L₄) lengths, preferably less than 65%, and/or wherein the fifth length (L₅) is within a range of 1-30 mm, and/or wherein the first (L₁), second (L₂), third (L₃), and fourth (L₄) lengths are equal.

8. The package according to any one of claims 5-7, wherein a stacking direction (SD) of each one of the at least one stack (201, 202) of absorbent articles (300) is perpendicular to the first and second side walls (106, 107) of the bag (100) and/or wherein the peripheral wall (103) of the bag (100) comprises an overlap seam (140) extending from the bottom (101) of the bag (100) to the top (102) of the bag (100), wherein the overlap seam (140) is formed at the front wall (104) or at the rear wall (105) of the bag (100).

9. The package according to any one of the preceding claims, wherein the bag (100) comprises at least one weakening line (161) for opening the bag (100), and preferably wherein the at least one weakening line (161) comprises a perforation line or a score line.

10. The package according to claim 9 when dependent on at least claim 5, wherein the at least one weakening line (161) is formed in or adjacent to at least one of said adhered portions (111, 121, 112, 122, 130).

11. The package according to any one of the preceding claims, wherein the bag (100) comprises at least one notch (160, 160') for tearing open the bag (100).

12. The package according to claim 11 when dependent on at least claim 5, wherein the at least one notch (160, 160') is formed in or adjacent to at least one of said adhered portions (111, 121, 112, 122, 130).

13. The package according to any one of the preceding claims, wherein each one of the at least one stack (201, 202) of absorbent articles (300) comprises a plurality of folded and individually wrapped absorbent articles (300), and preferably wherein a wrapper (301) of each individually wrapped absorbent article (300) comprises a sealed wrapper portion (302) facing upward within the bag (100).

14. The package according to any one of the preceding claims, wherein the sealed bottom (101) of the bag (100) is formed by folding and adhering a portion (119) of the paper material to form a flat and rectangular bottom (101), and/or wherein the bag (100) comprises an air displacement hole at or close to the bottom (101) of the bag (100) for allowing air to escape from the bag (100).

15. The package according to any one of the preceding claims, wherein the at least one stack (201, 202) of absorbent articles (300) comprises two stacks (201, 202) of absorbent articles (300), and/or wherein the bag (100) is opaque and the absorbent articles (300), such as a wrapper (301) of individually packed absorbent articles, cannot be seen through the bag (100).

## Patentansprüche

1. Verpackung (1) mit einem Beutel (100) und mindestens einem Stapel absorbierender Artikel (200), die in dem Beutel (100) angeordnet sind, wobei der Beutel (100) aus einem Papiermaterial hergestellt ist,
wobei der Beutel (100) einen versiegelten Boden (101), eine versiegelte Oberseite (102) und eine Umfangswand (103) aufweist, die sich zwischen dem Boden (101) und der Oberseite (102) erstreckt, wobei die Oberseite (101) durch aneinander Verkleben von inneren Oberseitenflächenabschnitten (113, 114, 115, 116, 117, 118, 123, 124, 125, 126) der Umfangswand (103) gebildet ist, wobei der Beutel (100) eine Klebeschicht (150) umfasst, die auf den inneren Flächenabschnitten (113, 114, 115, 116, 117, 118, 123, 124, 125, 126) der Umfangswand (103) vorgesehen ist, wobei die Klebeschicht (150) zum aneinander Verkleben der inneren Flächenabschnitte (113, 114, 115, 116, 117, 118, 123, 124, 125, 126) verwendet wird, wobei die Klebeschicht (150) ein Flächengewicht im Bereich von 28 bis 45 g/ m² aufweist.

2. Verpackung gemäß Anspruch 1, wobei das Flächengewicht der Klebeschicht (150) in einem Bereich von 30 bis 40 g/m², vorzugsweise 34 bis 38 g/m², liegt und/oder wobei das Verhältnis zwischen dem Flächengewicht des Papiermaterials und dem Flächengewicht der Klebeschicht (15) in einem Bereich von 1,6 bis 2,5, vorzugsweise 1,8 bis 2,2, liegt.

3. Verpackung gemäß einem der vorstehenden Ansprüche, wobei die Klebeschicht (150) eine Schmelzklebstoff-Klebeschicht ist.

4. Verpackung gemäß einem der vorstehenden Ansprüche, wobei das Papiermaterial ein Flächengewicht zwischen 50 und 90 g/ m² aufweist, gemessen gemäß der Norm ISO 536.

5. Verpackung (1) gemäß einem der vorstehenden Ansprüche, wobei die Umfangswand (103) eine Vorderwand (104), eine gegenüberliegende Rückwand (105) und eine erste und zweite Seitenwand (106, 107) umfasst, die die Vorder- und Rückwand (104, 105) verbinden, wobei die versiegelte Oberseite (102) durch aneinander Verkleben der inneren Oberseitenflächenabschnitte (113, 114, 115, 116, 117, 118, 123, 124, 125, 126) der Umfangswand (103) gebildet ist, sodass:
- ein erster Oberseitenabschnitt (113) der Vorderwand (104) an einen benachbarten ersten Oberseitenabschnitt (123) der zweiten Seitenwand (107) geklebt ist, um einen ersten geklebten Abschnitt (111) zu bilden,
- ein zweiter Oberseitenabschnitt (114) der Vorderwand (104) an einem benachbarten ersten Oberseitenabschnitt (124) der ersten Seitenwand (106) geklebt ist, um einen zweiten geklebten Abschnitt (112) zu bilden,
- ein erster Oberseitenabschnitt (115) der Rückwand (105) an einem benachbarten zweiten Oberseitenabschnitt (125) der zweiten Seitenwand (107) geklebt ist, um einen dritten geklebten Abschnitt (121) zu bilden,
- ein zweiter Oberseitenabschnitt (116) der Rückwand (105) an einem benachbarten zweiten Oberseitenabschnitt (126) der ersten Seitenwand (106) geklebt ist, um einen vierten geklebten Abschnitt (122) zu bilden, und
- ein dritter Oberseitenabschnitt (117) der Vorderwand (104) an einem dritten Oberseitenabschnitt (118) der Rückwand (105) geklebt ist, um einen fünften geklebten Abschnitt (130) zu bilden.

6. Verpackung (1) gemäß Anspruch 5, wobei der erste geklebte Abschnitt (111) eine erste Länge (L₁) aufweist, der zweite geklebte Abschnitt (112) eine zweite Länge (L₂) aufweist, der dritte geklebte Abschnitt (121) eine dritte Länge (L₃) aufweist, der vierte geklebte Abschnitt (122) eine vierte Länge (L₄) aufweist und der fünfte geklebte Abschnitt eine fünfte Länge (L₅) aufweist, wobei die fünfte Länge (L₅) kleiner ist als jede der ersten (L₁), zweiten (L₂), dritten (L₃) und vierten (L₄) Längen.

7. Verpackung gemäß Anspruch 6, wobei die fünfte Länge (L₅) weniger als 75 % einer kürzesten der ersten (L₁), zweiten (L₂), dritten (L₃) und vierten (L₄) Längen, vorzugsweise weniger als 65 %, ist und/oder wobei die fünfte Länge (L₅) in einem Bereich von 1 bis 30 mm liegt, und/oder wobei die erste (L₁), zweite (L₂), dritte (L₃) und vierte (L₄) Länge gleich sind.

8. Verpackung gemäß einem der Ansprüche 5 bis 7, wobei eine Stapelrichtung (SD) jedes einzelnen von dem mindestens einen Stapel (201, 202) von absorbierenden Artikeln (300) senkrecht zu der ersten und zweiten Seitenwand (106, 107) des Beutels (100) ist und/oder wobei die Umfangswand (103) des Beutels (100) eine Überlappungsnaht (140) aufweist, die sich vom Boden (101) des Beutels (100) bis zur Oberseite (102) des Beutels (100) erstreckt, wobei die Überlappungsnaht (140) an der Vorderwand (104) oder an der Rückwand (105) des Beutels (100) ausgebildet ist.

9. Verpackung gemäß einem der vorstehenden Ansprüche, wobei der Beutel (100) mindestens eine Schwächungslinie (161) zum Öffnen des Beutels (100) umfasst, und wobei die mindestens eine Schwächungslinie (161) vorzugsweise eine Perforationslinie oder eine Ritzlinie umfasst.

10. Verpackung gemäß Anspruch 9, wenn sie von mindestens Anspruch 5 abhängt, wobei die mindestens eine Schwächungslinie (161) in oder neben mindestens einem der verklebten Abschnitte (111, 121, 112, 122, 130) ausgebildet ist.

11. Verpackung gemäß einem der vorstehenden Ansprüche, wobei der Beutels (100) mindestens eine Kerbe (160, 160') zum Aufreißen des Beutels (100) aufweist.

12. Verpackung gemäß Anspruch 11, wenn sie von mindestens Anspruch 5 abhängt, wobei die mindestens eine Kerbe (160, 160') in oder neben mindestens einem der verklebten Abschnitte (111, 121, 112, 122, 130) ausgebildet ist.

13. Verpackung gemäß einem der vorstehenden Ansprüche, wobei jeder von dem mindestens einen Stapel (201, 202) von absorbierenden Artikeln (300) eine Vielzahl von gefalteten und einzeln umhüllten absorbierenden Artikeln (300) umfasst, und vorzugsweise, wobei eine Umhüllung (301) jedes einzeln verpackten absorbierenden Artikels (300) einen versiegelten Umhüllungsabschnitt (302) umfasst, der innerhalb des Beutels (100) nach oben zeigt.

14. Verpackung gemäß einem der vorstehenden Ansprüche, wobei der versiegelte Boden (101) des Beutels (100) durch Falten und Verkleben eines Abschnitts (119) des Papiermaterials gebildet wird, um einen flachen und rechteckigen Boden (101) zu bilden, und/oder wobei der Beutel (100) eine Luftverdrängungsöffnung am oder nahe dem Boden (101) des Beutels (100) aufweist, damit Luft aus dem Beutel (100) entweichen kann.

15. Verpackung gemäß einem der vorstehenden Ansprüche, wobei der mindestens eine Stapel (201, 202) von absorbierenden Artikeln (300) zwei Stapel (201, 202) von absorbierenden Artikeln (300) umfasst, und/oder wobei der Beutel (100) undurchsichtig ist und die absorbierenden Artikel (300), wie beispielsweise eine Umhüllung (301) von einzeln verpackten absorbierenden Artikeln, durch den Beutel (100) nicht sichtbar sind.

## Revendications

1. Emballage (1) comprenant un sac (100) et au moins une pile d'articles absorbants (200) disposés dans le sac (100), le sac (100) étant constitué d'un matériau de papier,
où le sac (100) a un fond scellé (101), un haut scellé (102) et une paroi périphérique (103) s'étendant entre le fond (101) et le haut (102), où le haut (101) a été formé en faisant adhérer des parties supérieures de surface interne (113, 114, 115, 116, 117, 118, 123, 124, 125, 126) de la paroi périphérique (103) les unes aux autres,
où le sac (100) comprend une couche d'adhésion (150) prévue sur les parties de surface interne (113, 114, 115, 116, 117, 118, 123, 124, 125, 126) de la paroi périphérique (103), ladite couche d'adhésion (150) étant utilisée pour faire adhérer lesdites parties de surface interne (113, 114, 126) les unes aux autres, où la couche d'adhésion (150) a un poids de base compris dans une plage de 28 à 45 g/m².

2. Emballage selon la revendication 1, où le poids de base de la couche d'adhésion (150) est compris dans une plage de 30 à 40 g/m², de préférence de 34 à 38 g/m², et/ou où un rapport entre un poids de base du matériau de papier et le poids de base de la couche d'adhésion (15) est compris dans une plage de 1,6 à 2,5, de préférence de 1,8 à 2,2.

3. Emballage selon l'une quelconque des revendications précédentes, où la couche d'adhésion (150) est une couche d'adhésion thermofusible.

4. Emballage selon l'une quelconque des revendications précédentes, où le matériau de papier a un poids de base compris entre 50 et 90 g/m² tel que mesuré selon la norme ISO 536.

5. Emballage (1) selon l'une quelconque des revendications précédentes, où la paroi périphérique (103) comprend une paroi avant (104), une paroi arrière opposée (105) et une première et deuxième paroi latérale (106, 107) reliant les parois avant et arrière (104, 105),
où le haut scellé (102) a été formé en faisant adhérer les parties supérieures de surface interne (113, 114, 115, 116, 117, 118, 123, 124, 125, 126) de la paroi périphérique (103) les unes aux autres de telle sorte que :
- une première partie supérieure (113) de la paroi avant (104) est adhérée à une première partie supérieure adjacente (123) de la deuxième paroi latérale (107) pour former une première partie adhérée (111),
- une deuxième partie supérieure (114) de la paroi avant (104) est adhérée à une première partie supérieure adjacente (124) de la première paroi latérale (106) pour former une deuxième partie adhérée (112),
- une première partie supérieure (115) de la paroi arrière (105) est adhérée à une deuxième partie supérieure adjacente (125) de la deuxième paroi latérale (107) pour former une troisième partie adhérée (121),
- une deuxième partie supérieure (116) de la paroi arrière (105) est adhérée à une deuxième partie supérieure adjacente (126) de la première paroi latérale (106) pour former une quatrième partie adhérée (122), et
- une troisième partie supérieure (117) de la paroi avant (104) est adhérée à une troisième partie supérieure (118) de la paroi arrière (105) pour former une cinquième partie adhérée (130).

6. Emballage (1) selon la revendication 5, où la première partie adhérée (111) a une première longueur (L₁), la deuxième partie adhérée (112) a une deuxième longueur (L₂), la troisième partie adhérée (121) a une troisième longueur (L₃), la quatrième partie adhérente (122) a une quatrième longueur (L₄), et la cinquième partie adhérente a une cinquième longueur (L₅), où la cinquième longueur (L₅) est inférieure à chacune de la première (L₁), la deuxième (L₄), la troisième (L₃) et la quatrième (L₄) longueur.

7. Emballage selon la revendication 6, où la cinquième longueur (L₅) est inférieure à 75 % de la plus courte de la première (L₁), la deuxième (L₂), la troisième (L₃) et la quatrième (L₄) longueur, de préférence inférieure à 65 %, et/ou où la cinquième longueur (L₅) est comprise dans une plage de 1 à 30 mm, et/ou
où la première (L₁), la deuxième (L₂), la troisième (L₃) et la quatrième (L₄) longueur sont égales.

8. Emballage selon l'une quelconque des revendications 5 à 7, où une direction d'empilement (SD) de chacune de l'au moins une pile (201, 202) d'articles absorbants (300) est perpendiculaire à la première et à la deuxième paroi latérale (106, 107) du sac (100) et/ou où la paroi périphérique (103) du sac (100) comprend une couture de chevauchement (140) s'étendant du fond (101) du sac (100) jusqu'au haut (102) du sac (100),
où la couture de chevauchement (140) est formée au niveau de la paroi avant (104) ou au niveau de la paroi arrière (105) du sac (100).

9. Emballage selon l'une quelconque des revendications précédentes, où le sac (100) comprend au moins une ligne d'affaiblissement (161) pour ouvrir le sac (100), et de préférence où l'au moins une ligne d'affaiblissement (161) comprend une ligne de perforation ou une ligne de rupture.

10. Emballage selon la revendication 9 lorsqu'il dépend d'au moins la revendication 5, où l'au moins une ligne d'affaiblissement (161) est formée dans ou adjacente à au moins une desdites parties adhérées (111, 121, 112, 122, 130).

11. Emballage selon l'une quelconque des revendications précédentes, où le sac (100) comprend au moins une encoche (160, 160') pour déchirer le sac (100).

12. Emballage selon la revendication 11, lorsqu'il dépend d'au moins la revendication 5, où l'au moins une encoche (160, 160') est formée dans ou adjacente à au moins une desdites parties adhérées (111, 121, 112, 122, 130).

13. Emballage selon l'une quelconque des revendications précédentes, où chacune de l'au moins une pile (201, 202) d'articles absorbants (300) comprend une pluralité d'articles absorbants (300) pliés et enveloppés individuellement, et de préférence où une enveloppe (301) de chaque article absorbant enveloppé individuellement (300) comprend une partie d'enveloppe scellée (302) tournée vers le haut à l'intérieur du sac (100).

14. Emballage selon l'une quelconque des revendications précédentes, où le fond scellé (101) du sac (100) est formé en pliant et en faisant adhérer une partie (119) du matériau de papier pour former un fond plat et rectangulaire (101), et/ou où le sac (100) comprend un trou de déplacement d'air au niveau ou à proximité du fond (101) du sac (100) pour permettre à l'air de s'échapper du sac (100).

15. Emballage selon l'une quelconque des revendications précédentes, où l'au moins une pile (201, 202) d'articles absorbants (300) comprend deux piles (201, 202) d'articles absorbants (300), et/ou où le sac (100) est opaque et les articles absorbants (300), tels qu'une enveloppe (301) d'articles absorbants emballés individuellement, ne peuvent pas être vus à travers le sac (100).
